(19)

Europäisches
Patentamt
European
Patent Office
Office européen
des brevets

(11) **EP 4 238 651 A1**

(12) **EUROPEAN PATENT APPLICATION**
published in accordance with Art. 153(4) EPC

(43) Date of publication:
**06.09.2023 Bulletin 2023/36**

(21) Application number: **21885214.3**

(22) Date of filing: **27.10.2021**

(51) International Patent Classification (IPC):
**B01J 37/03** (2006.01)      **B01J 23/755** (2006.01)
**B01J 23/78** (2006.01)      **C07C 209/48** (2006.01)
**C07C 211/27** (2006.01)

(52) Cooperative Patent Classification (CPC):
**B01J 23/755; B01J 23/78; B01J 23/80;
B01J 23/883; B01J 37/03; B01J 37/18;
C07C 209/48; C07C 211/27**

(86) International application number:
**PCT/CN2021/126727**

(87) International publication number:
**WO 2022/089487 (05.05.2022 Gazette 2022/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.10.2020  CN 202011164914**

(71) Applicants:
• **China Petroleum & Chemical Corporation
  Beijing 100728 (CN)**
• **Shanghai Research Institute of Petrochemical
  Technology, SINOPEC
  Shanghai 201208 (CN)**

(72) Inventors:
• **TU, Yunbao
  Shanghai 201208 (CN)**

• **ZONG, Hongyuan
  Shanghai 201208 (CN)**
• **LIU, Zhongneng
  Shanghai 201208 (CN)**
• **XU, Xiaoqing
  Shanghai 201208 (CN)**
• **BAI, Xue
  Shanghai 201208 (CN)**
• **LIU, Xu
  Shanghai 201208 (CN)**
• **FU, Wei
  Shanghai 201208 (CN)**
• **WANG, Yanhong
  Shanghai 201208 (CN)**

(74) Representative: **karo IP
karo IP Patentanwälte
Kahlhöfer Rößler Kreuels PartG mbB
Postfach 32 01 02
40416 Düsseldorf (DE)**

(54) **CATALYST FOR PREPARING DIAMINE BY HYDROGENATION OF DINITRILE, AND
PREPARATION METHOD THEREFOR AND APPLICATION THEREOF**

(57)    The present invention discloses a catalyst for producing dibasic amine by hydrogenation of dibasic nitrile, which comprises the following components or reaction product thereof: a) an active component, wherein the active component comprises Ni and/or an oxide thereof; b) an auxiliary, wherein the auxiliary comprises one or more of Mg, Cu, Co, Zn, Zr, Mo and/or oxides thereof; C) support, wherein the relative content of $\alpha$-NiO in the catalyst is less than 2.0 a.u.. The present invention also discloses a process for producing dibasic amine by hydrogenation of dibasic nitrile. The catalyst and the process of the present invention greatly reduce the generation of excessive hydrogenation by-products and improve the overall selectivity of target products.

EP 4 238 651 A1

**Figure 1**

**Description**

**Technical Field**

The present invention belongs to the field of dibasic amine preparation, and in particular relates to a catalyst for producing dibasic amine by hydrogenation of dibasic nitrile and a process for preparing the same, a process for producing dibasic amine by hydrogenation of dibasic nitrile and use thereof.

**Background Technology**

m-Xylylenediamine (meta-benzenedimethanamine) can be used as raw material for epoxy resin curing agent. The curing agent made from m-xylylenediamine can be used as a modified epoxy resin curing agent due to containing aromatic aliphatic amine. Its characteristics include the accelerated curing rate at room temperature; good heat resistance, water resistance, and reagent resistance; and good wetting curing ability and surface gloss. It is widely used in coatings, adhesives, and electronic-grade products.

m-Xylylenediamine is also used as raw material for synthesizing MX nylon and its derivatives, especially MXD6, which is prepared together with adipic acid. This nylon is characterized by high strength and elasticity over a high temperature range, high deformation temperature, low thermal expansion rate (equivalent to alloys). It is suitable for the precision molding and can be used for high-temperature baking and coating. The produced film has high transparency and oxygen impermeability, and is suitable for food packaging. The produced fiber has a high strength.

m-Xylylenediamine can also be used as raw material for polyurethane resin. This can be used to prepare meta-bis(isocyanatomethyl)benzene, which can further synthesize polyurethane resin. This resin is comparable to hexamethylene diisocyanate and has better yellowing resistance than the latter. It can be used in light colored coatings with high coating hardness and low toxicity, and can also be used in synthetic leather.

At present, m-xylylenediamine is mostly produced by catalytic hydrogenation of isophthalonitrile.

Patent application No. CN200680036084.8 discloses a process flow for preparing MXDA (m-xylylenediamine) from IPN (isophthalonitrile) by fixed-bed continuous hydrogenation method. At 170-200°C, IPN is melted and mixed in a liquid form with liquid ammonia and recycled material and dissolved (60°C). Under the conditions of 60-130°C and 150-200 bar, in the fixed-bed reactor under the catalysis of Mn-doped non-supported Co catalyst, the per-pass conversion rate is >99%, and the selectivity is >92%.

Patent application No. CN200680035201.9 discusses using the product MXDA recycle material as the IPN solvent, and dissolving at 55-70°C. The technical processes provided in patent applications CN201010150757.0 and CN201010150725.0 mainly comprise: adding the modified Raney Ni catalyst into the stirred reactor in advance, then pumping isophthalonitrile and a ternary mixed solvent (an aromatic hydrocarbon, a low-carbon alcohol, an aliphatic halogenated derivative) and a secondary amine as inhibitor. After dissolved, the reaction is performed at 40-120°C under 2-10 MPa in a stirred tank to (intermittently) produce MXDA through the hydrogenation.

However, in the prior art, there are still problems such as large catalyst consumption, unsatisfactory product selectivity, and intermittent operation of high-pressure autoclave. Therefore, it is necessary to develop new catalysts with high activity and high selectivity and realize continuous industrial production.

**Summary of the Invention**

The technical problem to be solved by the present invention is to provide a new catalyst for preparing m-xylylenediamine in high selectivity and a process for preparing the same, in response to the low selectivity in producing m-xylylenediamine by hydrogenation of isophthalonitrile in the prior art.

Therefore, the first aspect of the present invention provides a catalyst for producing dibasic amine by hydrogenation of dibasic nitrile, which comprises the following components or reaction product thereof:

a) an active component, wherein the active component comprises Ni and/or an oxide thereof;
b) an auxiliary, wherein the auxiliary comprises one or more of Mg, Cu, Co, Zn, Zr, Mo and/or oxides thereof;
c) a support;

wherein the relative content of $\alpha$-NiO in the catalyst is less than 2.0 a.u..

According to some embodiments of the present invention, the relative content of $\alpha$-NiO in the catalyst is less than 1.5 a.u., preferably less than 0.2 a.u..

According to some embodiments of the present invention, the auxiliary comprises one or more of Cu, Co, Zr, Mo and/or oxides thereof.

The content in "relative content" of the present invention is not an absolute concept of content, but refers to a relative content of a substance determined by comparison of the integral areas of the peaks in the same graph in the same coordinate system, where the size of the integral area of the peak represents the content of the substance in the catalyst. Specifically, for the relative content of α-NiO of the present invention (using a.u. as the unit), the same graph in the same coordinate system refers to the $H_2$-TPR diagram of the catalyst, where the abscissa is the temperature in degrees Celsius (°C), and the ordinate is the signal value of the TCD (thermal conductivity detector) (it is a quantitative value, for example expressed in the form of a percentage or a decimal). For the relative content of α-NiO of the present invention, when measuring $H_2$-TPR, a catalyst in a fully oxidized state is used, and the amount of the catalyst is 50 mg.

In the present invention, a.u. is an abbreviation of arbitrary unit, which refers to a relative value and is dimensionless.

According to some embodiments of the present invention, the total weight of the catalyst is 100wt%.

The content of the active component is 5-70 wt%, preferably 10-60 wt%, more preferably 15-50 wt%; the content of the auxiliary is 0.1-60 wt%, preferably 1-50 wt%, more preferably 5-45 wt%; the content of said support is 10-90 wt%, preferably 15-80 wt%, more preferably 20-70 wt%.

According to some other examples of the present invention, based on the parts by weight, the content of the active component is 10-60 parts, preferably 15- 55 parts; the content of the auxiliary is 0.1-120 parts, preferably 0.2-90 parts; the content of said support is 0.1-45 parts, preferably 1-35 parts.

According to some embodiments of the present invention, said support is at least one of alumina, silica and zeolite, preferably alumina.

According to some embodiments of the present invention, said support is a support that has been treated at a high temperature of not less than 500°C.

The second aspect of the present invention provides a process for preparing the catalyst according to the first aspect of the present invention, wherein the process comprises the following steps:

1) treating a support at a temperature not lower than 500°C, and then performing the first contact of a solution of auxiliary salt (i.e. a salt of the auxiliary), a solution of first precipitant, the support and water to produce a modified support;

2) performing the second contact of a solution of nickel salt, a solution of second precipitant, the modified support obtained from step 1) and water, filtering, and calcining to produce a catalyst.

According to some embodiments of the present invention, in step 1), the solution of auxiliary salt and the solution of first precipitant are simultaneously added to the water containing the support to perform the first contact.

According to some embodiments of the present invention, in step 2), the solution of auxiliary salt and the solution of second precipitant are simultaneously added to the water containing the modified support obtained from step 1) to perform the second contact.

According to some embodiments of the present invention, in step 1), the resulting solution in the first contact is controlled to the endpoint pH of 6.0-10.0, for example 6.0-8.0.

According to some embodiments of the present invention, in step 2), the resulting solution in the second contact is controlled to the endpoint pH of 6.0-10.0, for example 6.0-8.0.

According to some embodiments of the present invention, the temperature of said first contact is 50-90°C, in some embodiments, said temperature is 70°C. According to some embodiments of the present invention, the time of said first contact is 3-6 hours.

According to some embodiments of the present invention, the temperature of said second contact is 50-90°C.

According to some embodiments of the present invention, the time of said second contact is 3-6 hours.

According to some embodiments of the present invention, said auxiliary salt is selected from one or more of $Mg(NO_3)_2$, $Cu(NO_3)_2$, $Co(NO_3)_2$, $Zn(NO_3)_2$, $Zr(NO_3)_4$, and $(NH_4)_2MoO_4$, preferably selected from one or more of $Cu(NO_3)_2$, $Zr(NO_3)_4$, $(NH_4)_2MoO_4$ and $Co(NO_3)_2$.

According to some embodiments of the present invention, the auxiliary salt may be a hydrate of salt, for example one or more of $Mg(NO_3)_2 \cdot 6H_2O$, $Cu(NO_3)_2 \cdot 3H_2O$, $Co(NO_3)_2 \cdot 6H_2O$, $Zn(NO_3)_2 \cdot 6H_2O$ and $Zr(NO_3)_4 \cdot 5H_2O$.

According to some embodiments of the present invention, said first precipitant is selected from one or more of sodium hydroxide, potassium hydroxide, sodium carbonate, sodium bicarbonate and ammonia water, preferably sodium hydroxide and/or ammonia water.

According to some embodiments of the present invention, said nickel salt is nickel sulphate and/or nickel nitrate, preferably nickel nitrate.

According to some embodiments of the present invention, said second precipitant is selected from one or more of sodium hydroxide, potassium hydroxide, sodium carbonate, sodium bicarbonate and ammonia water, preferably selected from one or more of sodium hydroxide, sodium carbonate and sodium bicarbonate.

According to some embodiments of the present invention, in step 1), said solution of auxiliary salt has a concentration of 0.1-1.5 mol/L, preferably 0.3-1.2 mol/L.

According to some embodiments of the present invention, in step (1), said solution of first precipitant has a concentration of 0.4-2.0 mol/L, preferably 0.6-1.6 mol/L.

According to some embodiments of the present invention, in step (1), the content of said support in water is 5-100 g/L, for example 20-80 g/L, or 5-20 g/L, e.g. 8-15 g/L.

According to some embodiments of the present invention, in step 2), said solution of nickel salt has a concentration of 0.2-1.5 mol/L, preferably 0.5-1.2 mol/L.

According to some embodiments of the present invention, in step 2), said solution of second precipitant has a concentration of 0.4-2.0 mol/L, preferably 0.6-1.5 mol/L.

According to some embodiments of the present invention, in step 2), the content of said modified support in water is 10-100 g/L, for example 20-85 g/L, or 10-30 g/L, e.g. 12-25 g/L.

According to some embodiments of the present invention, in step 2), the calcining is performed in an air atmosphere.

According to some embodiments of the present invention, in step 2), the calcining temperature is 300-600°C.

According to some embodiments of the present invention, in step 2), a solution of the nickel salt and a solution of the second precipitant are simultaneously added to the water containing the modified support under the condition of 50-90°C, and mixed, the mixed solution is controlled to the endpoint pH of 6.0-10.0, for example 6.0-8.0, stirred for 3-6 hours, filtered, washed, dried, and calcined at 300-600°C in an oxygen-containing atmosphere (such as air atmosphere or pure oxygen) to produce a catalyst.

The third aspect of the present invention provides a process for producing dibasic amine by hydrogenation of dibasic nitrile, which comprises contacting the dibasic nitrile with hydrogen gas in the presence of the catalyst according to the first aspect of the present invention or the catalyst prepared with the process according to the second aspect of the present invention to react to produce the dibasic amine.

According to some embodiments of the present invention, the reaction temperature is 50-120°C, preferably 60-80°C.

According to some embodiments of the present invention, said reaction pressure is 4.0-15.0 MPa, for example, 4.0-12.0 MPa, or 6.0-10.0 MPa.

According to some embodiments of the present invention, the liquid hourly space velocity of said reaction is 1-12 hr$^{-1}$, preferably 2-10 hr$^{-1}$.

According to some embodiments of the present invention, the molar ratio of hydrogen gas to dibasic nitrile of said reaction is 3:1-70:1, preferably 5:1-20:1.

According to some embodiments of the present invention, said isophthalonitrile is dissolved in liquid ammonia. In some embodiments, the mass fraction of isophthalonitrile is 10%. In some embodiments, the mass fraction of said liquid ammonia is 90%.

The fourth aspect of the present invention provides use of the catalyst according to the first aspect of the present invention, or the catalyst prepared with the process according to the second aspect of the present invention, or the process according to the third aspect of the present invention in producing dibasic amine by hydrogenation of dibasic nitrile, especially producing m-xylylenediamine by hydrogenation of isophthalonitrile.

Generally speaking, the present invention provides the following technical solutions:

1. A catalyst, which comprises:

   a) an active component, wherein the active component comprises an oxide of Ni;
   b) an auxiliary, preferably, the auxiliary comprises one or more of oxides of Mg, Cu, Co, Zn, Zr, and Mo, more preferably the auxiliary comprises one or more of oxides of Cu, Co, Zr, and Mo;
   c) a support;

wherein, the relative content of $\alpha$-NiO in the catalyst is less than 2.0 a.u., for example less than or equal to 1.5 a.u., further for example less than or equal to 0.2 a.u..

2. A catalyst in a fully oxidized state, which comprises:

   a) an active component, wherein the active component comprises an oxide of Ni;
   b) an auxiliary, preferably, the auxiliary comprises one or more of oxides of Mg, Cu, Co, Zn, Zr, and Mo, more preferably the auxiliary comprises one or more of oxides of Cu, Co, Zr, and Mo;
   c) a support;

wherein, the content of $\alpha$-NiO in the catalyst is less than 55wt%, less than 50wt%, less than 40wt%, less than 30wt%, less than 20wt%, less than 10wt%, for example less than 5 wt%.

The fully oxidized state described herein refers to a catalyst obtained by calcining the corresponding catalyst in an oxygen-containing atmosphere (such as air atmosphere or pure oxygen) at a certain calcining temperature (e.g. 300-600° C) for a long enough time (e.g. 4 hours, 8 hours, 12 hours, 24 hours, 48 hours, 96 hours, 1 week, 2 weeks, 4 weeks or longer), and the fully oxidized state can also be achieved by other methods, not limited to the above-mentioned calcining methods.

3. A catalyst, which comprises:

    a) an active component, wherein the active component is Ni and/or an oxide thereof;
    b) an auxiliary, preferably, the auxiliary comprises one or more of Mg, Cu, Co, Zn, Zr, Mo and/or oxides thereof, more preferably the auxiliary comprises one or more of Cu, Co, Zr, Mo and/or oxides thereof;
    c) a support;

wherein, when said catalyst is in its fully oxidized state, the content of $\alpha$-NiO in the catalyst is less than 55wt%, less than 50wt%, less than 40wt%, less than 30wt%, less than 20wt%, less than 10wt%, for example less than 5 wt%.

4. A catalyst, which comprises:

    a) an active component, wherein the active component is Ni and/or an oxide thereof;
    b) an auxiliary, preferably, the auxiliary comprises one or more of Mg, Cu, Co, Zn, Zr, Mo and/or oxides thereof, more preferably the auxiliary comprises one or more of Cu, Co, Zr, Mo and/or oxides thereof;
    c) a support;

wherein, when said catalyst is in its fully oxidized state, the relative content of $\alpha$-NiO in the catalyst is less than 2.0 a.u., for example less than or equal to 1.5 a.u., further for example less than or equal to 0.2 a.u..

5. The catalyst according to any of the aforementioned technical solutions, which is characterized in that based on the parts by weight,

    the content of the active component is 10-60 parts by weight, for example 15-55 parts by weight;
    the content of the auxiliary is 0.1-120 parts by weight, for example 0.2-90 parts by weight;
    the content of said support is 0.1-45 parts by weight, for example 1-35 parts by weight;
    or
    based on the total weight of the catalyst being 100wt%,
    the content of the active component is 5-70 wt%, preferably 10-60 wt%, more preferably 15-50 wt%;
    the content of the auxiliary is 0.1-60 wt%, preferably 1-50 wt%, more preferably 5-45 wt%;
    the content of said support is 10-90wt%, preferably 15-80wt%, more preferably 20-70wt%;
    or
    based on the total weight of the catalyst being 100wt%,
    the content of the active component is 30-35wt%;
    the content of the auxiliary is 15-50 wt%;
    the content of said support is 15-60 wt%;
    preferably, the auxiliary is Co, Mo or Zr, more preferably the auxiliary is Co.

6. The catalyst according to any of the aforementioned technical solutions, which is characterized in that said support is at least one of alumina, silica and zeolite, for example alumina; preferably, said support is a support that has been treated at a temperature of not less than 500°C.

7. The catalyst according to any of the aforementioned technical solutions, which is characterized in that said support comprises alumina, wherein based on the weight of alumina, the proportions of $\alpha$, $\beta$, $\gamma$, $\delta$, $\theta$-alumina are 0.1-99%, 0.1-99%, 0.1-99%, 0.1-99%, 0.1-99% respectively, preferably 10-95%, 1-70%, 2-90%, 5-90%, 3-80% respectively.

8. The catalyst according to any of the aforementioned technical solutions, which is characterized in that the catalyst is a catalyst for producing dibasic amine by hydrogenation of dibasic nitrile.

9. The catalyst according to any of the aforementioned technical solutions, which is characterized in that the relative content of $\alpha$-NiO in the catalyst is greater than 0.0001 a.u., for example greater than 0.001 a.u., further for example greater than 0.01 a.u.; or the content of $\alpha$-NiO in the catalyst is greater than 0.0001 wt%, for example greater than 0.001 wt%, further for example greater than 0.01 wt%.

10. A process for preparing the catalyst according to any one of the aforementioned technical solutions, which is characterized in that said process comprises the following steps:

    1) performing the first contact of a solution of auxiliary salt, a solution of first precipitant, a support and water to produce a modified support;
    2) performing the second contact of a solution of nickel salt, a solution of second precipitant, the modified support obtained from step 1) and water, filtering, and calcining to produce a catalyst,

for example, in step 1), the solution of auxiliary salt and the solution of first precipitant are simultaneously added to the water containing the support to perform the first contact, and/or in step 2), the solution of nickel salt and the solution of second precipitant are simultaneously added to the water containing the modified support obtained from step 1) to perform the second contact.

11. The process according to technical solution 7, which is characterized in that in step 1), the used support is a support that has been treated at a temperature of not less than 500°C, preferably the used support is a support that has been treated at a temperature of higher than 500°C.

12. The process according to any one of technical solutions 10-11, which is characterized in that the resulting solution in the first contact and the resulting solution in the second contact are controlled to the endpoint pH of 6.0-10.0, for example 6.0-8.0,

for example, the temperature of said first contact and/or second contact is 50-90°C, and/or the time of said first contact and/or second contact is 3-6 hours.

13. The process according to any of technical solutions 10-12, which is characterized in that said auxiliary salt is selected from one or more of $Mg(NO_3)_2$, $Cu(NO_3)_2$, $Co(NO_3)_2$, $Zn(NO_3)_2$, $Zr(NO_3)_4$, $(NH_4)_2MoO_4$, $Mg(NO_3)_2 \cdot 6H_2O$, $Cu(NO_3)_2 \cdot 3H_2O$, $Co(NO_3)_2 \cdot 6H_2O$, $Zn(NO_3)_2 \cdot 6H_2O$ and $Zr(NO_3)_4 \cdot 5H_2O$, for example, selected from one or more of $Cu(NO_3)_2$, $Zr(NO_3)_4$, $(NH_4)_2MoO_4$ and $Co(NO_3)_2$;

and/or said first precipitant is selected from one or more of sodium hydroxide, potassium hydroxide, sodium carbonate, sodium bicarbonate and ammonia water, preferably, the first precipitant is carbon-free, more preferably, the first precipitant is sodium hydroxide and/or ammonia water;
and/or the nickel salt is nickel sulphate and/or nickel nitrate, for example nickel nitrate;
and/or said second precipitant is selected from one or more of sodium hydroxide, potassium hydroxide, sodium carbonate, sodium bicarbonate and ammonia water, preferably, said second precipitant is carbonaceous, more preferably said second precipitant is sodium carbonate and/or sodium bicarbonate.

14. The process according to any of technical solutions 10-13, which is characterized in that in step (1), said solution of auxiliary salt has a concentration of 0.1-1.5 mol/L, for example 0.3-1.2 mol/L; and/or said solution of first precipitant has a concentration of 0.4-2.0 mol/L, for example 0.6-1.6 mol/L; and/or the content of said support in water is 5-100 g/L, for example 20-80 g/L, or 5-20 g/L, e.g. 8-15 g/L.

15. The process according to any of technical solutions 10-14, which is characterized in that in step 2), said solution of nickel salt has a concentration of 0.2-1.5 mol/L, for example 0.5-1.2 mol/L; and/or said solution of second precipitant has a concentration of 0.4-2.0 mol/L, for example 0.6-1.5 mol/L, and/or the content of said support in water is 10-100 g/L, for example 20-85 g/L, or 10-30 g/L, e.g. 12-25 g/L.

16. A process for producing dibasic amine by hydrogenation of dibasic nitrile, which comprises contacting the dibasic nitrile with hydrogen gas in the presence of the catalyst according to any of technical solutions 1-9 or the catalyst prepared with the process according to any of technical solutions 10-15 to react to produce the dibasic amine, for example the molar ratio of said hydrogen gas to the dibasic nitrile is 3:1-70:1, for example 5:1-20:1.

For example, the reaction temperature is 50-120°C, for example 60-80°C; and/or said reaction pressure is 4.0-15.0 MPa, for example 4.0-12.0 MPa, further for example 6.0-10.0 MPa; and/or the liquid hourly space velocity of said reaction is 1-12 $hr^{-1}$, for example 2-10 $hr^{-1}$.

In the present invention, there is no special limitation on dibasic amines and dibasic nitriles, wherein the dibasic nitrile can be aliphatic $C_4$-$C_{24}$ dibasic nitriles, such as adiponitrile, pimelonitrile, suberonitrile, nonanedinitrile, decanedinitrile, undecanedinitrile, dodecanedinitrile, tridecanedinitrile, tetradecanedinitrile, pentadecanedinitrile, hexadecanedinitrile, heptadecanedinitrile, or octadecanedinitrile, or aromatic $C_6$-$C_{18}$ dibasic nitriles, such as phthalonitrile, isophthalonitrile, or terephthalonitrile;

The dibasic amine can be aliphatic $C_4$-$C_{24}$ dibasic amine such as hexanediamine, heptanediamine, octanediamine, nonanediamine, decanediamine, undecanediamine, dodecanediamine, tridecanediamine, tetradecanediamine, pentadecanediamine, hexadecanediamine, heptadecanediamine, or octadecanediamine, or aromatic $C_6$-$C_{18}$ dibasic amine such as ortho-benzenedimethanamine, meta-benzenedimethanamine, or para-benzenedimethanamine.

17. Use of the catalyst according to any of technical solutions 1-9 or the catalyst prepared with the process according to any one of technical solutions 10-15 or the process according to technical solutions 13 in producing dibasic amine by hydrogenation of dibasic nitrile, especially in producing aliphatic $C_4$-$C_{24}$ dibasic amine (such as hexanediamine, heptanediamine, octanediamine, nonanediamine, decanediamine, undecanediamine, dodecanediamine, tridecanediamine, tetradecanediamine, pentadecanediamine, hexadecanediamine, heptadecanediamine, or octadecanediamine) or aromatic $C_6$-$C_{18}$ dibasic amine (such as ortho-benzenedimethanamine, meta-benzenedimethanamine, or para-benzenedimethanamine) by hydrogenation of aliphatic $C_4$-$C_{24}$ dibasic nitrile (such as adiponitrile, pimelonitrile, suberonitrile, nonanedinitrile, decanedinitrile, undecanedinitrile, dodecanedinitrile, tridecanedinitrile, tetradecanedinitrile, pentadecanedinitrile, hexadecanedinitrile, heptadecanedinitrile, or octadecanedinitrile) or aromatic $C_6$-$C_{18}$ dibasic nitrile (such as phthalonitrile, isophthalonitrile, or terephthalonitrile).

For the supported nickel-based catalyst, there are usually three forms of NiO: free amorphous α-NiO, β1-NiO weakly interacting with the support, and β2-NiO strongly interacting with the support. Too much free amorphous α-NiO in the catalyst will lead to more excessive hydrogenation side reactions to generate hydrogenolysis by-products such as 3-methylbenzylamine and m-xylene.

Herein, the content of α-NiO refers to the weight percent or the relative content of α-NiO relative to the total amount of nickel species as NiO in the catalyst when the catalyst is converted into the fully oxidized state.

The present invention aims at the influence of different NiO types of nickel-based catalysts on the selectivity of target products, and provides a catalyst and process for producing diamine by hydrogenation of dibasic nitrile, especially for producing m-xylylenediamine by hydrogenation of isophthalonitrile. The catalyst and the process of the present invention greatly reduce the generation of excessive hydrogenation by-products and improve the overall selectivity of target products.

**Brief description of the drawings**

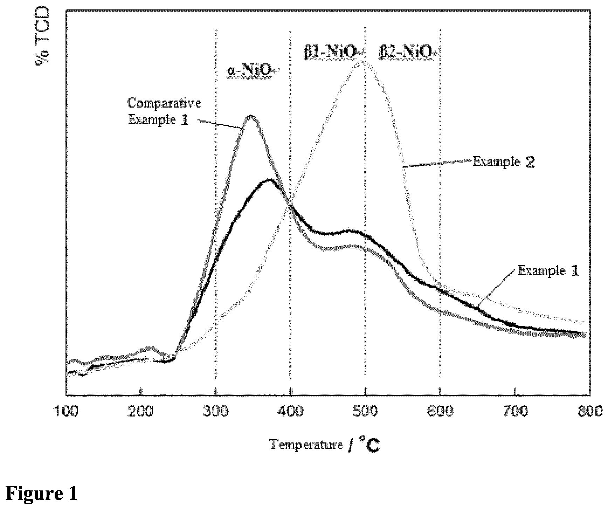

Figure 1 shows the $H_2$-TPR diagrams of the catalysts prepared in Examples 1 and 2 and Comparative Example 1. wherein (1) α-NiO (300-400°C): attributed to free amorphous NiO species on the surface; (2) β1-NiO (400-500°C): attributed to the NiO species weakly interacting with the support; (3) β2-NiO (500-600°C): attributed to the NiO species strongly interacting with the support.

**Detailed description**

In order to make the present invention easier to understand, the present invention will be described in detail below in conjunction with the embodiments and accompanying drawings. These embodiments are only used to illustrate the present invention and should not be regarded as limiting the scope of the present invention. Those who do not indicate the specific conditions in the examples are carried out according to the conventional conditions or the conditions suggested by the manufacturer. The used reagents or instruments, for which the manufacturers are not indicated herein, are commercially available or obtained by conventional methods.

The end points of the ranges and any values disclosed herein are not limited to the precise ranges or values, and these ranges or values should be understood to include the values close to these ranges or values. For numerical ranges, the endpoints of the ranges to each other, the endpoints of the ranges and the individual point values, and the individual point values to each other can be combined with each other to give one or more new numerical ranges, and these new numerical ranges should be construed as specifically disclosed herein.

In the concept used in the present invention, the conversion ratio and the selectivity for producing m-xylylenediamine by hydrogenation of isophthalonitrile are calculated by the following equations:

$$\text{IPN conversion ratio} = \frac{n_{IPN,1} - n_{IPN,2}}{n_{IPN,1}} \times 100\%$$

$$\text{MXDA selectivity} = \frac{n_{MXDA,2}}{n_{IPN,1} - n_{IPN,2}} \times 100\%$$

in which, n: the amount of substance, in unit of mol; IPN: isophthalonitrile; MXDA: m-xylylenediamine; 1: raw materials; 2: product.

Test method:

1. The test method for the relative content of different types of NiO: the area integration of the $H_2$-TPR curve (the ordinate is %TCD, and the abscissa is temperature) is performed, and the relative content of different types of NiO is calculated based on the hydrogen consumption of the sample, and the unit is a.u. (arbitrary unit). The size of the peak area actually represents the relative content of different types of NiO, and is used for the mutual comparison of NiO between different samples.

In the following examples, the isophthalonitrile used is of the industrial grade and dissolved in liquid ammonia, the mass fraction of isophthalonitrile is 10%, and the mass fraction of liquid ammonia is 90%; the volume fraction of the used hydrogen gas is 99.9%.

The constitution of the catalyst is measured according to the X-ray fluorescence method (with reference to "Petrochemical Analysis Method (RIPP Test Method)", edited by Yang Cuiding et al., Science Press, published in 1990).

Example 1

(1) Catalyst preparation

Preparation of modified support: the auxiliary salt $Co(NO_3)_2$ was made into a solution I with a concentration of 0.8 mol/L, sodium hydroxide was made into a solution II with a concentration of 1.0 mol/L, and the precursor of the aluminum hydroxide support (pseudo-boehmite) was pretreated at a high temperature of 500°C and placed in 1L of water. At 70°C, solution I and solution II were co-currently precipitated, and the endpoint pH was controlled to 7.0. The aging while stirring was performed for 3-6 hours to produce a modified alumina support.

Preparation of catalyst: nickel nitrate was made into a solution III with a concentration of 0.8 mol/L, sodium carbonate was made into a solution IV with a concentration of 1.2 mol/L, and the obtained modified alumina support (50g) was placed in 1L of water. At 70°C, solution III and solution IV were co-currently precipitated, and the endpoint pH was controlled to 7.5. The aging while stirring was performed for 4 hours, and then filtering, washing, drying, and calcining in the air atmosphere at 500°C for 6 hours were performed to produce a catalyst. In the resulting catalyst, the weight of CoO was 0.75g, the weight of the active component as nickel oxide was 5.25g, and the weight of the alumina support was 9.0g. The $H_2$-TPR diagram was shown in Figure 1, and the results were shown in Table 1.

(2) Catalyst reduction

15g of the obtained catalyst was taken, which contained CoO (0.75g) in the catalyst components. The catalyst was loaded to 15mL, and reduced with pure hydrogen gas at 500°C for 24 hours to produce a reduced catalyst.

(3) Producing m-xylylenediamine by hydrogenation of isophthalonitrile

A solution of isophthalonitrile in liquid ammonia (3000 mL, the mass fraction of isophthalonitrile was 10%, the mass fraction of liquid ammonia was 90%) and a pure hydrogen gas (the volume fraction of hydrogen gas was 99.9%) were used as raw materials, the used amount of the reduced catalyst was 15g, and the hydrogenation test was performed under the following conditions: the reaction temperature was 80°C, the reaction pressure was 8.0 MPa, the hydrogen gas/isophthalonitrile molar ratio was 5:1, and the liquid hourly space velocity was 10 hr$^{-1}$. The reaction results were shown in Table 1. The IPN conversion rate was 99.9%, the MXDA selectivity was 97.1%. The content of 3-methylbenzylamine was 0.32% (measured by liquid chromatography).

Example 2

The catalyst of this example was prepared by the preparation method of the catalyst in Example 1, except that a different content of Co was used in the catalyst: the weight of CoO in the catalyst was 2.25g. The $H_2$-TPR diagram was shown in Figure 1, and the results were shown in Table 1.

(1) Catalyst reduction:

15g of the catalyst was taken, which contained CoO (2.25g) in the catalyst. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.9%, the MXDA selectivity was 98.3%. The content of 3-methylbenzylamine was 0.13%.

Example 3

The catalyst of this example was prepared by the preparation method of the catalyst in Example 1, except that a different content of Co was used in the catalyst: the weight of CoO in the catalyst was 0.5g. The result of the relative content of $\alpha$-NiO was shown in Table 1.

(1) Catalyst reduction:

15g of the catalyst was taken, which contained CoO (0.5g) in the catalyst. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.9%, the MXDA selectivity was 96.4%. The content of 3-methylbenzylamine was 0.42%.

Example 4

The catalyst of this example was prepared by the preparation method of the catalyst in Example 1, except that a different content of Co was used in the catalyst: the weight of CoO in the catalyst was 4.5g. The result of the relative content of $\alpha$-NiO was shown in Table 1.

(1) Catalyst reduction:

15g of the catalyst was taken, which contained CoO (4.5g) in the catalyst. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.9%, the MXDA selectivity was 98.4%. The content of 3-methylbenzylamine was 0.11%.

Example 5

The catalyst of this example was prepared by the preparation method of the catalyst in Example 1, except that a different content of Co was used in the catalyst: the weight of CoO in the catalyst was 7.5g. The result of the relative content of $\alpha$-NiO was shown in Table 1.

(1) Catalyst reduction:

15g of the catalyst was taken, which contained CoO (7.5g) in the catalyst. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.9%, the MXDA selectivity was 98.5%. The content of 3-methylbenzylamine was 0.09%.

Example 6

The catalyst of this example was prepared by the preparation method of the catalyst in Example 2, except that a different auxiliary salt was used: it was $Zr(NO_3)_4$. The result of the relative content of $\alpha$-NiO in the catalyst was shown in Table 1.

(1) Catalyst reduction:

15g of the catalyst was taken. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.9%, the MXDA selectivity was 98.0%. The content of 3-methylbenzylamine was 0.17%.

Example 7

The catalyst of this example was prepared by the preparation method of the catalyst in Example 2, except that a different auxiliary salt was used: it was $Mg(NO_3)_2$. The result of the relative content of $\alpha$-NiO in the catalyst was shown in Table 1.

(1) Catalyst reduction:

15g of the catalyst was taken. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.9%, the MXDA selectivity was 97.8%. The content of 3-methylbenzylamine was 0.23%.

Example 8

The catalyst of this example was prepared by the preparation method of the catalyst in Example 2, except that a different auxiliary salt was used: it was $Cu(NO_3)_2$. The result of the relative content of $\alpha$-NiO in the catalyst was shown in Table 1.

(1) Catalyst reduction:

15g of the catalyst was taken. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.7%, the MXDA selectivity was 96.9%. The content of 3-methylbenzylamine was 0.36%.

Example 9

The catalyst of this example was prepared by the preparation method of the catalyst in Example 2, except that a different auxiliary salt was used: it was $Zn(NO_3)_2$. The result of the relative content of $\alpha$-NiO in the catalyst was shown in Table 1.

(1) Catalyst reduction:

15g of the catalyst was taken. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.8%, the MXDA selectivity was 96.4%. The content of 3-methylbenzylamine was 0.41%.

Example 10

The catalyst of this example was prepared by the preparation method of the catalyst in Example 2, except that a different auxiliary salt was used: it was $(NH_4)_2MoO_4$. The result of the relative content of $\alpha$-NiO in the catalyst was shown in Table 1.

(1) Catalyst reduction:

15g of the catalyst was taken. The catalyst was loaded to 15mL, and reduced with pure hydrogen gas at 500°C for 24 hours to produce a reduced catalyst.

(2) Catalytic Hydrogenation of Catalyst

The method was the same as that in Example 1, and the reaction results were shown in Table 1. The IPN conversion rate was 99.9%, the MXDA selectivity was 98.1%. The content of 3-methylbenzylamine was 0.19%.

Example 11

(1) Catalyst preparation

The solution of the auxiliary salt $Co(NO_3)_2$, the solution III of nickel nitrate, and the solution of the precipitant in Example 1 were added together to the water containing the aluminum hydroxide support having been treated at a high temperature of 500°C, and the endpoint pH of the solution was controlled to 7.0 so that the nickel salt, the auxiliary salt and the precipitant were precipitated together on the support alumina. The result of the relative content of $\alpha$-NiO in the catalyst was shown in Table 1.

(2) Catalyst reduction

15g of the obtained catalyst was taken. The catalyst was loaded to 15mL, and reduced with pure hydrogen gas at 500°C for 24 hours to produce a reduced catalyst.

(3) Producing m-xylylenediamine by hydrogenation of isophthalonitrile

A solution of isophthalonitrile in liquid ammonia (3000 mL, the mass fraction of isophthalonitrile was 10%, the mass fraction of liquid ammonia was 90%) and a pure hydrogen gas (the volume fraction of hydrogen gas was 99.9%) were used as raw materials, the used amount of the catalyst was 15g, and the hydrogenation test was performed under the following conditions: the reaction temperature was 80°C, the reaction pressure was 8.0 MPa, the hydrogen gas/isophthalonitrile molar ratio was 5:1, and the liquid hourly space velocity was 10 $hr^{-1}$. The reaction results were shown in Table 1. The IPN conversion rate was 99.9%, the MXDA selectivity was 95.2%. The content of 3-methylbenzylamine was 0.49%.

Comparative Example 1

(1) Catalyst preparation

The process was identical to that of Example 1 except that the solution of auxiliary salt $Co(NO_3)_2$ was not added during the preparation of the catalyst. The $H_2$-TPR diagram was shown in Figure 1, and the results were shown in Table 1.

(2) Catalyst reduction

15g of the obtained catalyst was taken, which contained no CoO in the catalyst components. The catalyst was loaded to 15mL, and reduced with pure hydrogen gas at 500°C for 24 hours.

(3) Producing m-xylylenediamine by hydrogenation of isophthalonitrile

A solution of isophthalonitrile in liquid ammonia (3000 mL, the mass fraction of isophthalonitrile was 10%, the mass fraction of liquid ammonia was 90%) and a pure hydrogen gas (the volume fraction of hydrogen gas was 99.9%) were used as raw materials, the used amount of the reduced catalyst was 15g, and the hydrogenation test was performed under the following conditions: the reaction temperature was 80°C, the reaction pressure was 8.0 MPa, the hydrogen gas/isophthalonitrile molar ratio was 5:1, and the liquid hourly space velocity was 10 $hr^{-1}$. The reaction results were shown in Table 1. The IPN conversion rate was 99.9%, the MXDA selectivity was 95.6%. The content of 3-methylbenzylamine was 0.58%.

Comparative Example 2

(1) Catalyst preparation

The process was identical to that of Example 1 except that the alumina support had not been treated at a high temperture of 500°C. The result of the relative content of $\alpha$-NiO in the catalyst was shown in Table 1.

(2) Catalyst reduction

15g of the obtained catalyst was taken. The catalyst was loaded to 15mL, and reduced with pure hydrogen gas at 500°C for 24 hours to produce a reduced catalyst.

(3) Producing m-xylylenediamine by hydrogenation of isophthalonitrile

A solution of isophthalonitrile in liquid ammonia (3000 mL, the mass fraction of isophthalonitrile was 10%, the mass fraction of liquid ammonia was 90%) and a pure hydrogen gas (the volume fraction of hydrogen gas was 99.9%) were used as raw materials, the used amount of the catalyst was 15g, and the hydrogenation test was performed under the following conditions: the reaction temperature was 80°C, the reaction pressure was 8.0 MPa, the hydrogen gas/isophthalonitrile molar ratio was 5:1, and the liquid hourly space velocity was 10 hr$^{-1}$. The reaction results were shown in Table 1. The IPN conversion rate was 99.8%, the MXDA selectivity was 94.9%. The content of 3-methylbenzylamine was 0.45%.

It should be noted that the above-mentioned embodiments are only used to explain the present invention, and do not constitute any limitation to the present invention. The present invention has been described with reference to exemplary embodiments, but it is to be understood that the words used therein are words of description and explanation, rather than words of limitation. The present invention may be modified within the scope of the claims of the present invention as specified, and may be modified without departing from the scope and spirit of the present invention. Although the present invention described herein refers to the specific methods, materials and embodiments, it is not intended to be limited to the specific examples disclosed therein, but rather, the present invention extends to all other methods and applications having the same function.

Table 1

| No. | Catalyst weight (g) | Alumina support weight (g) | Active component Ni (g, as oxide) | Auxiliary (g, as oxide) | α-NiO relative content (a.u.) | Content of α-NiO in Ni species (wt%, as NiO) | IPN conversion /% | MXDA selectivity /% | 3-methyl benzyl amine content/% |
|---|---|---|---|---|---|---|---|---|---|
| Ex. 1 | 15 | 9.0 | 5.25 | 0.75 | 1.4 | 50.5 | 99.9 | 97.1 | 0.32 |
| Ex. 2 | 15 | 7.5 | 5.25 | 2.25 | 0.1 | 1.9 | 99.9 | 98.3 | 0.13 |
| Ex. 3 | 15 | 9.25 | 5.25 | 0.5 | 1.6 | 53.8 | 99.9 | 96.4 | 0.42 |
| Ex. 4 | 15 | 5.25 | 5.25 | 4.5 | 0.1 | 1.7 | 99.9 | 98.4 | 0.11 |
| Ex. 5 | 15 | 3.0 | 4.5 | 7.5 | 0.05 | 1.1 | 99.9 | 98.5 | 0.09 |
| Ex. 6 | 15 | 7.5 | 5.25 | 2.25 | 0.2 | 4.8 | 99.9 | 98.0 | 0.17 |
| Ex. 7 | 15 | 7.5 | 5.25 | 2.25 | 0.5 | 11.2 | 99.9 | 97.8 | 0.23 |
| Ex. 8 | 15 | 7.5 | 5.25 | 2.25 | 1.4 | 49.6 | 99.7 | 96.9 | 0.36 |
| Ex. 9 | 15 | 7.5 | 5.25 | 2.25 | 1.5 | 51.2 | 99.8 | 96.4 | 0.41 |
| Ex. 10 | 15 | 7.5 | 5.25 | 2.25 | 0.3 | 7.4 | 99.9 | 98.1 | 0.19 |
| Ex. 11 | 15 | 9.0 | 5.25 | 0.75 | 1.7 | 54.9 | 99.9 | 95.2 | 0.49 |
| Comp. Ex. 1 | 15 | 9.0 | 6.0 | 0 | 2.1 | 61.3 | 99.9 | 95.6 | 0.58 |
| Comp. Ex. 2 | 15 | 9.0 | 5.25 | 0.75 | 2.0 | 59.4 | 99.8 | 94.9 | 0.45 |

**Claims**

1. A catalyst, which comprises:

   a) an active component, wherein the active component comprises an oxide of Ni;
   b) an auxiliary, preferably, the auxiliary comprises one or more of oxides of Mg, Cu, Co, Zn, Zr, and Mo, more preferably the auxiliary comprises one or more of oxides of Cu, Co, Zr, and Mo;
   c) a support;

   wherein, the relative content of $\alpha$-NiO in the catalyst is less than 2.0 a.u., for example less than 1.5 a.u., further for example less than 0.2 a.u..

2. The catalyst according to claim 1, which is **characterized in that** based on the parts by weight,

   the content of the active component is 10-60 parts, for example 15-55 parts;
   the content of the auxiliary is 0.1-120 parts, for example 0.2-90 parts;
   The content of said support is 0.1-45 parts, for example 1-35 parts.

3. The catalyst according to claim 1, which is **characterized in that** said support is at least one of alumina, silica and zeolite, for example alumina; preferably, said support is a support that has been treated at a temperature of not less than 500°C.

4. The catalyst according to claim 1, which is **characterized in that** said support comprises alumina, wherein based on the weight of alumina, the proportions of $\alpha$, $\beta$, $\gamma$, $\delta$, $\theta$-alumina are 0.1-99%, 0.1-99%, 0.1-99%, 0.1-99%, 0.1-99% respectively, preferably 10-95%, 1-70%, 2-90%, 5-90%, 3-80% respectively.

5. The catalyst according to claim 1, which is **characterized in that** the catalyst is a catalyst for producing dibasic amine by hydrogenation of dibasic nitrile.

6. The catalyst according to claim 1, which is **characterized in that** the relative content of $\alpha$-NiO in the catalyst is greater than 0.0001 a.u., for example greater than 0.001 a.u., further for example greater than 0.01 a.u.; or the content of $\alpha$-NiO in the catalyst is greater than 0.0001 wt%, for example greater than 0.001 wt%, further for example greater than 0.01 wt%.

7. A process for preparing the catalyst according to any one of the preceding claims, which is **characterized in that** said process comprises the following steps:

   1) performing the first contact of a solution of auxiliary salt, a solution of first precipitant, a support and water to produce a modified support;
   2) performing the second contact of a solution of nickel salt, a solution of second precipitant, the modified support obtained from step 1) and water, filtering, and calcining to produce a catalyst,

   for example, in step 1), the solution of auxiliary salt and the solution of first precipitant are simultaneously added to the water containing the support to perform the first contact, and/or in step 2), the solution of nickel salt and the solution of second precipitant are simultaneously added to the water containing the modified support obtained from step 1) to perform the second contact.

8. The process according to claim 7, which is **characterized in that** in step 1), the used support is a support that has been treated at a temperature of not less than 500°C, preferably the used support is a support that has been treated at a temperature of higher than 500°C.

9. The process according to claim 7, which is **characterized in that** the resulting solution in the first contact and the resulting solution in the second contact are controlled to the endpoint pH of 6.0-10.0, for example 6.0-8.0, for example, the temperature of said first contact and/or second contact is 50-90°C, and/or the time of said first contact and/or second contact is 3-6 hours.

10. The process according to claim 7, which is **characterized in that** said auxiliary salt is selected from one or more of $Mg(NO_3)_2$, $Cu(NO_3)_2$, $Co(NO_3)_2$, $Zn(NO_3)_2$, $Zr(NO_3)_4$, $(NH_4)_2MoO_4$, $Mg(NO_3)_2 \cdot 6H_2O$, $Cu(NO_3)_2 \cdot 3H_2O$,

$Co(NO_3)_2 \cdot 6H_2O$, $Zn(NO_3)_2 \cdot 6H_2O$ and $Zr(NO_3)_4 \cdot 5H_2O$, for example selected from one or more of $Cu(NO_3)_2$, $Zr(NO_3)_4$, $(NH_4)_2MoO_4$ and $Co(NO_3)_2$;

and/or said first precipitant is selected from one or more of sodium hydroxide, potassium hydroxide, sodium carbonate, sodium bicarbonate and ammonia water, preferably, the first precipitant is carbon-free, more preferably, the first precipitant is sodium hydroxide and/or ammonia water;
and/or the nickel salt is nickel sulphate and/or nickel nitrate, for example nickel nitrate;
and/or said second precipitant is selected from one or more of sodium hydroxide, potassium hydroxide, sodium carbonate, sodium bicarbonate and ammonia water, preferably, said second precipitant is carbonaceous, more preferably said second precipitant is sodium carbonate and/or sodium bicarbonate.

11. The process according to claim 7, which is **characterized in that** in step 1), said solution of auxiliary salt has a concentration of 0.1-1.5 mol/L, for example 0.3-1.2 mol/L; and/or said solution of first precipitant has a concentration of 0.4-2.0 mol/L, for example 0.6-1.6 mol/L; and/or the content of said support in water is 5-100 g/L, for example 20-80 g/L, or 5-20 g/L, e.g. 8-15 g/L.

12. The process according to claim 7, which is **characterized in that** in step 2), said solution of nickel salt has a concentration of 0.2-1.5 mol/L, for example 0.5-1.2 mol/L; and/or said solution of second precipitant has a concentration of 0.4-2.0 mol/L, for example 0.6-1.5 mol/L, and/or the content of said support in water is 10-100 g/L, for example 20-85 g/L, or 10-30 g/L, e.g. 12-25 g/L.

13. A process for producing dibasic amine by hydrogenation of dibasic nitrile, comprising in the presence of the catalyzt according to any one of claims 1-6 or the catalyst prepared with the process according to any one of claims 7-12, the dibasic nitrile is contacted with hydrogen gas to generate the dibasic amine, for example, the molar ratio of hydrogen gas to dibasic nitrile is 3:1-70:1, for example 5:1-20:1,
for example, the reaction temperature is 50-120°C, for example 60-80°C; and/or said reaction pressure is 4.0-15.0 MPa, for example 4.0-12.0 MPa, further for example 6.0-10.0 MPa; and/or the liquid hourly space velocity of said reaction is 1-12 $hr^{-1}$, for example 2-10 $hr^{-1}$.

14. Use of the catalyst according to any one of claims 1-6 or the catalyst prepared with the process according to any one of claims 7-12 or the process according to claim 13 in producing dibasic amine by hydrogenation of dibasic nitrile, especially in producing aliphatic $C_4$-$C_{24}$ dibasic amine (such as hexanediamine, heptanediamine, octanediamine, nonanediamine, decanediamine, undecanediamine, dodecanediamine, tridecanediamine, tetradecanediamine, pentadecanediamine, hexadecanediamine, heptadecanediamine, or octadecanediamine) or aromatic $C_6$-$C_{18}$ dibasic amine (such as ortho-benzenedimethanamine, meta-benzenedimethanamine, or para-benzenedimethanamine) by hydrogenation of aliphatic $C_4$-$C_{24}$ dibasic nitrile (such as adiponitrile, pimelonitrile, suberonitrile, nonanedinitrile, decanedinitrile, undecanedinitrile, dodecanedinitrile, tridecanedinitrile, tetradecanedinitrile, pentadecanedinitrile, hexadecanedinitrile, heptadecanedinitrile, or octadecanedinitrile) or aromatic $C_6$-$C_{18}$ dibasic nitrile (such as phthalonitrile, isophthalonitrile, or terephthalonitrile).

**Figure 1**

## INTERNATIONAL SEARCH REPORT

| International application No. |
|---|
| **PCT/CN2021/126727** |

| A. | CLASSIFICATION OF SUBJECT MATTER |
|---|---|

B01J 37/03(2006.01)i; B01J 23/755(2006.01)i; B01J 23/78(2006.01)i; C07C 209/48(2006.01)i; C07C 211/27(2006.01)i

According to International Patent Classification (IPC) or to both national classification and IPC

| B. | FIELDS SEARCHED |
|---|---|

Minimum documentation searched (classification system followed by classification symbols)

B01J 23/-、 B01J 37/-、 C07C 209/-、 C07C 211/-

Documentation searched other than minimum documentation to the extent that such documents are included in the fields searched

Electronic data base consulted during the international search (name of data base and, where practicable, search terms used)

CNABS, CNTXT, CNKI, VEN, web of science: 镍, Ni, 载体, 沉淀, 腈, 氢, 胺, nickel, carrier, deposit, precipitation, nitrile, hydroge+

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 107805203 A (SHANGHAI INSTITUTE OF TECHNOLOGY) 16 March 2018 (2018-03-16) description, paragraphs 5-16 | 1-14 |
| A | CN 106807395 A (DALIAN INSTITUTE OF CHEMICAL PHYSICS, CHINESE ACADEMY OF SCIENCES) 09 June 2017 (2017-06-09) entire document | 1-14 |
| A | EP 0340848 A2 (UNILEVER NV et al.) 08 November 1989 (1989-11-08) entire document | 1-14 |
| A | CN 101670289 A (FEIXIANG CHEMICALS (ZHANGJIAGANG) CO., LTD. et al.) 17 March 2010 (2010-03-17) entire document | 1-14 |
| A | EP 3061743 A1 (BASF SE) 31 August 2016 (2016-08-31) entire document | 1-14 |
| A | US 2003120115 A1 (BASF AG) 26 June 2003 (2003-06-26) entire document | 1-14 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **02 December 2021** | **26 January 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

| International application No. |
| --- |
| **PCT/CN2021/126727** |

| Patent document cited in search report | | | Publication date (day/month/year) | Patent family member(s) | | | Publication date (day/month/year) |
| --- | --- | --- | --- | --- | --- | --- | --- |
| CN | 107805203 | A | 16 March 2018 | None | | | |
| CN | 106807395 | A | 09 June 2017 | None | | | |
| EP | 0340848 | A2 | 08 November 1989 | NO | 891855 | D0 | 05 May 1989 |
| | | | | NO | 891855 | A | 07 November 1989 |
| | | | | NO | 891855 | L | 07 November 1989 |
| | | | | NO | 170148 | B | 09 June 1992 |
| | | | | NO | 170148 | C | 16 September 1992 |
| | | | | AT | 93510 | T | 15 September 1993 |
| | | | | JP | H0211541 | A | 16 January 1990 |
| | | | | DE | 68908597 | D1 | 30 September 1993 |
| | | | | DE | 68908597 | T2 | 23 December 1993 |
| | | | | NL | 8801192 | A | 01 December 1989 |
| | | | | NL | 186511 | B | 16 July 1990 |
| | | | | NL | 186511 | C | 17 December 1990 |
| | | | | ES | 2042967 | T3 | 16 December 1993 |
| | | | | DE | 3914875 | A1 | 16 November 1989 |
| | | | | DE | 3914875 | C2 | 22 November 1990 |
| | | | | EP | 0340848 | A3 | 31 October 1990 |
| | | | | EP | 0340848 | B1 | 25 August 1993 |
| CN | 101670289 | A | 17 March 2010 | None | | | |
| EP | 3061743 | A1 | 31 August 2016 | None | | | |
| US | 2003120115 | A1 | 26 June 2003 | US | 6790996 | B2 | 14 September 2004 |
| | | | | AT | 411976 | T | 15 November 2008 |
| | | | | DE | 50212927 | D1 | 04 December 2008 |
| | | | | DE | 10152135 | A1 | 30 April 2003 |
| | | | | JP | 2003192647 | A | 09 July 2003 |
| | | | | EP | 1306365 | A2 | 02 May 2003 |
| | | | | EP | 1306365 | A3 | 15 October 2003 |
| | | | | EP | 1306365 | B1 | 22 October 2008 |

Form PCT/ISA/210 (patent family annex) (January 2015)

**REFERENCES CITED IN THE DESCRIPTION**

**Patent documents cited in the description**

- CN 200680036084
- CN 200680035201
- CN 201010150757
- CN 201010150725

**Non-patent literature cited in the description**

- Petrochemical Analysis Method (RIPP Test Method). Science Press, 1990